Europäisches Patentamt

European Patent Office

Office européen des brevets

(11) Veröffentlichungsnummer : **0 477 747 B1**

(12)

# EUROPÄISCHE PATENTSCHRIFT

(45) Veröffentlichungstag der Patentschrift :
09.02.94 Patentblatt 94/06

(51) Int. Cl.$^5$ : **A61K 7/46**

(21) Anmeldenummer : **91115812.9**

(22) Anmeldetag : **18.09.91**

(54) Verfahren zur Herstellung eines Riechstoffes.

(30) Priorität : **27.09.90 DE 4030562**

(43) Veröffentlichungstag der Anmeldung :
**01.04.92 Patentblatt 92/14**

(45) Bekanntmachung des Hinweises auf die
Patenterteilung :
**09.02.94 Patentblatt 94/06**

(84) Benannte Vertragsstaaten :
**AT BE CH DE DK ES FR GB GR IT LI LU NL SE**

(56) Entgegenhaltungen :
DE-A- 2 219 168
DE-A- 2 235 466
PATENT ABSTRACTS OF JAPAN, unexamined
applications, C Sektion, Band 3, Nr. 14, 8.
Februar 1979 THE PATENT OFFICE JAPA-
NESE GOVERNMENT Seite 50 C 36

(73) Patentinhaber : HOECHST
AKTIENGESELLSCHAFT
D-65926 Frankfurt (DE)

(72) Erfinder : Bruns, Klaus, Prof.Dr.
Notburgaweg 6
W-4150 Krefeld Traar (DE)
Erfinder : Gerke, Thomas, Dr.
Im Melchersfeld 6
W-4040 Neuss 1 (DE)
Erfinder : Bahrmann, Helmut, Dr.
Rohstrasse 48
W-4236 Hamminkeln 3 (DE)
Erfinder : Heymanns, Peter, Dr.
Kaulbachstrasse 4
W-4300 Essen 1 (DE)
Erfinder : Weber, Jürgen, Dr.
Bunsenstrasse 17
W-4200 Oberhausen 11 (DE)

EP 0 477 747 B1

## Beschreibung

Die vorliegende Erfindung betrifft die Herstellung eines Riechstoffes durch Umsetzung von Sassafrasöl mit Kohlenmonoxid und Wasserstoff in Gegenwart eines Rhodiumkatalysators. Durch diese Reaktion werden im Einsatzmaterial enthaltene, olefinisch ungesättigte Verbindungen hydroformyliert, d.h. in Aldehyde überführt. Das neue Verfahren ermöglicht die Herstellung eines Riechstoffes aus preiswert zur Verfügung stehenden Ausgangsstoffen.

Es ist bekannt, Safrol, 1,2-Methylendioxy-4-allylbenzol zu hydroformylieren. Nach der DE-A1-2 235 466 führt man diese Reaktion bei 70°C und einem Druck von 600 atü CO/H$_2$ (Volumenverhältnis 1 : 1) in Gegenwart von Rhodium, das in Form eines chlorhaltigen Cyclooctadien-Komplexes eingesetzt wird, als Katalysator durch. Das Reaktionsprodukt enthält im Verhältnis 53 : 47 4-(3,4-Methylendioxyphenyl)-butanal und 2-Methyl-3-(3,4-methylendioxyphenyl)-propanal. Durch Behandlung dieses Produktes mit Piperidinacetat läßt sich die Propanal-Verbindung gewinnen. Man kann das Reaktionsgemisch aber auch durch fraktionierte Destillation trennen und erhält dann beide Verbindungen, das substituierte Propanal und das substituierte Butanal in reiner Form.

In gleicher Weise kann auch Isosafrol, 1,2-Methylendioxy-4-propenylbenzol, hydroformyliert werden unter Bildung von 2-(3,4-Methylendioxyphenyl)-butanal (vgl. DE-Al-2 219 168).

Einen anderen Weg zur Hydroformylierung von Safrol und Isosafrol beschreitet das in der offengelegten japanischen Patentanmeldung 78/137963 beschriebene Verfahren.

Die Umsetzung erfolgt in Gegenwart von Rhodium, das dem Reaktionsgemisch als Acetat zugesetzt wird, bei 120°C und 230 kg/cm$^2$ Druck.

In der offengelegten japanischen Patentanmeldung 79/09271 werden die Verbindungen 4-(3,4-Methylendioxyphenyl)-butanal und 2-(3,4-Methylendioxyphenyl)-butanal als Riechstoffe beschrieben.

Die bekannten Verfahren zur Herstellung von Riechstoffen aus Safrol und Isosafrol gehen von den reinen Verbindungen als Einsatzstoffen aus und erfordern überdies aufwendige Maßnahmen zur Trennung der Hydroformylierungsgemische, um die Aldehydverbindungen mit den gewünschten Geruchseigenschaften zu isolieren. Sie sind infolgedessen aus wirtschaftlichen Gründen nicht zur Parfümierung von Massenprodukten wie Seifen oder Waschmittel geeignet.

Es bestand daher die Aufgabe, ein Verfahren zur Gewinnung von Riechstoffen zu entwickeln, das auf preiswerten Rohstoffen basiert, technisch einfach durchzuführen ist und hohe Ausbeuten ergibt.

Die Erfindung besteht in einem Verfahren zur Herstellung eines Riechstoffes. Es ist dadurch gekennzeichnet, daß man Sassafrasöl bei Drücken von 5 bis 50 MPa und Temperaturen von 70 bis 170°C in Gegenwart von Triphenylphosphan als Ligand enthaltenden Rhodiumcarbonyl-Komplexverbindungen zusammen mit überschüssigem Triphenylphosphan als Katalysator mit einem Gemisch aus Kohlenmonoxid und Wasserstoff umsetzt und das Reaktionsprodukt von Lösungsmittel und Katalysator abtrennt.

Aus der analytischen Untersuchung des Reaktionsproduktes geht hervor, daß seine Hauptbestandteile 4-(3,4-Methylendioxyphenyl)-butanal und 2-Methyl-3-(3,4-methylendioxyphenyl)-propanal sind.

Überraschenderweise kann zur Herstellung eines Riechstoffes mit einer frischen, fruchtigen Duftnote vom Typ Wassermelone anstelle von reinem Safrol Sassafrasöl, ein sehr preiswerter Ausgangsstoff, eingesetzt werden. Es war nicht vorauszusehen, daß die im Sassafrasöl enthaltenen Nebenbestandteile weder die Hydroformylierung stören, noch (auch in Form möglicher Reaktionsprodukte) die Riechstoffqualität des Reaktionsgemisches beeinträchtigen. Ebenso wenig war zu erwarten, daß die im wesentlichen aus zwei isomeren Aldehyden bestehende Mischung eine interessante Duftnote aufweist. Denn bisher war man bestrebt, durch gezielte Maßnahmen während der Synthese - z.B. Einsatz spezieller Katalysatoren - oder durch aufwendige Reinigungsoperationen, die Bildung des 4-(3,4-Methylendioxyphenyl)-butanals ("4-Butanal") zu unterdrücken oder entstandenes 4-Butanal möglichst vollständig abzutrennen. Diese Zielsetzung führte möglicherweise auch dazu, daß man den Einsatz des erfindungsgemäß verwendeten Katalysators bisher vermied, weil er die Isomerisierung, also die Bildung des 4-Butanals und des isomeren 2-(3,4-methylendioxyphenyl)-butanals, begünstigen soll. Schließlich überrascht auch, daß bis zu etwa 80 % des im Sassafrasöl enthaltenen Safrols zu Aldehyden umgesetzt wird, eine Ausbeute, die bei Anwendung der bekannten Verfahren unter Einsatz reinen Safrols, nicht annähernd erzielt wurde.

Das als Ausgangsstoff verwendete Sassafrasöl ist ein durch Wasserdampfdestillation aus der Wurzelrinde und dem Stammholz einer Lorbeerbaumart gewonnenes, gelbes bis rötlich gelbes ätherisches Öl. Sein wichtigster Inhaltsstoff ist mit einem Anteil von mindestens 80 % Safrol. Daneben finden sich in dem Sassafrasöl Phellandren, alpha-Pinen, D-Campfer, Eugenol und ein Gemisch verschiedener Sesquiterpene.

Zur Herstellung des Riechstoffes nach dem erfindungsgemäßen Verfahren kann das Sassafrasöl in der handelsüblichen Form, d.h. ohne Reinigung oder Trennung in die Bestandteile eingesetzt werden.

Ein wesentliches Merkmal des erfindungsgemäßen Verfahrens ist die Verwendung einer Rhodiumcarbonyl-Komplexverbindung als Katalysator. Diese Komplexverbindung enthält als einen Liganden Triphenylphosphan, das im Überschuß eingesetzt wird, d.h. in einer Menge, die über den zur Komplexbildung erforderlichen Anteil hinausgeht. Zweckmäßig setzt man je g-At Rhodium 10 bis 150 Mol Triphenylphosphan ein.

Rhodium wird, bezogen auf das im Reaktionsgemisch vorhandene Olefin, in einer Konzentration von 1 bis 100, insbesondere 5 bis 20 ppm angewandt.

Der Katalysator kann in präformierter Form eingesetzt werden. Es ist aber auch möglich, ihn in situ aus dem Metall oder einem Rhodiumsalz, sowie Wassergas und den Triphenylphosphanen zu bilden. Als Rhodiumsalze verwendet man zweckmäßig in organischen Medien lösliche Verbindungen. Bewährt haben sich Rhodiumacetat und Rhodiumsalze höherer Fettsäuren insbesondere Rhodium-2-ethylhexanoat.

Die Umsetzung des Sassafrasöls mit Kohlenmonoxid und Wasserstoff erfolgt bei Drücken von 5 bis 50 MPa, bevorzugt bei 20 bis 30 MPa. Die Reaktionstemperatur beträgt 70 bis 170°C, vorzugsweise 120 bis 140°C.

Kohlenmonoxid und Wasserstoff werden im allgemeinen in einem Volumenverhältnis von 1 : 4 bis 4 : 1, insbesondere 1 : 1 eingesetzt. Bezogen auf den Ausgangsstoff wendet man das Gasgemisch im Überschuß an, z.B. bis zur 100fachen molaren Menge.

Die Umsetzung des Sassafrasöls kann ohne Verwendung eines Lösungsmittels erfolgen. Es hat sich jedoch bewährt, das Einsatzmaterial in Gegenwart eines inerten Lösungsmittels zu hydroformylieren; geeignet sind Kohlenwasserstoffe wie Pentan, Hexan, Cyclohexan, Benzol, Toluol, Xylol, ferner Ether wie Tetrahydrofuran und Dioxan. Vorzugsweise wendet man Benzol, Toluol oder Xylol an.

Die Herstellung des neuen Riechstoffs kann kontinuierlich oder diskontinuierlich in einem Hochdruckreaktor erfolgen. Sassafrasöl wird zusammen mit dem präformierten Katalysator oder den Katalysatorausgangsstoffen Rhodiummetall oder Rhodiumverbindung und Triphenylphosphan im Reaktor vorgelegt und unter den obengenannten Bedingungen mit Kohlenmonoxid und Wasserstoff umgesetzt. Nach Beendigung der Umsetzung trennt man das Reaktionsprodukt z.B. durch fraktionierte Destillation von Lösungsmittel und Katalysator ab.

In den folgenden Beispielen wird das neue Verfahren beschrieben, es ist aber selbstverständlich nicht auf diese Ausführungsformen beschränkt.

Beispiel 1

In einem 14 l Rohrreaktor wird bei einer Temperatur von 130°C und einem Druck von 27 MPa eine Lösung von Sassafrasöl in Toluol (50 Gew.-% Sassafrasöl, bezogen auf die Lösung) mit Wasserstoff und Kohlenmonoxid ($H_2$ : CO = 1 : 1 in Vol.) umgesetzt. Als Katalysator findet Rhodium (als 2-Ethylhexanoat)/Triphenylphosphan im Molverhältnis 1 : 100 Anwendung, mit einer auf Sassafrasöl bezogenen Rhodiumkonzentration von 100 ppm. Der Durchsatz beträgt 0,22 Volumen Lösung/Reaktorvolumen . h.

Das Sassafrasöl wird vollständig umgesetzt. Es resultiert ein Rohprodukt, das, lösungsmittelfrei gerechnet, 89,6 Gew.-% Aldehyde enthält; nach gaschromatographischer Analyse hat es folgende Zusammensetzung:

| | |
|---|---|
| nicht identifizierte Komponenten | 7,8 Gew.-% |
| Safrol | 0,35 Gew.-% |
| 4-(3,4-Methylendioxyphenyl)-butanal | 55,3 Gew.-% |
| 2-Methyl-3-(3,4-Methylendioxyphenyl)-propanal | 33,8 Gew.-% |
| 2-(3,4-Methylendioxyphenyl)-butanal | 0,45 Gew.-% |
| Nachlauf | 2,3 Gew.-% |

Beispiel 2

Beispiel 1 wird bei einer Temperatur im Bereich von 140 bis 145°C unter sonst übereinstimmenden Bedingungen wiederholt. Das Rohprodukt hat nach gaschromatographischer Analyse folgende Zusammensetzung:

```
Toluol                                           47,3  Gew.-%

nicht identifizierte Komponenten                  4,2  Gew.-%

Safrol                                            0,2  Gew.-%

4-(3,4-Methylendioxyphenyl)-butanal              28,3  Gew.-%

2-Methyl-3-(3,4-Methylendioxy-
phenyl)-propanal                                 18,1  Gew.-%

2-(3,4-Methylendioxyphenyl)-butanal               1,2  Gew.-%

Nachlauf                                          0,7  Gew.-%
```

Es wird durch Dünnschichtverdampfung in zwei Stufen gereinigt. In der ersten Stufe erfolgt bei 125°C Manteltemperatur und 45 hPa Druck die Abtrennung von Lösungsmittel und Vorlauf (%-Angaben bezogen auf Einsatzgemisch)

```
Destillat (davon 93,7 Gew.-% Toluol)    44,8  Gew.-%

Rückstand                               53,3  Gew.-%

Kondensat (Kühlfalle)                    1,9  Gew.-%
```

In der zweiten Stufe wird der Rückstand der ersten Stufe bei 200°C Manteltemperatur und 4 hPa destilliert (%-Angaben bezogen auf Einsatzgemisch)

Destillat       88,0 Gew.-%
Rückstand       11,6 Gew.-%
Kondensat (Kühlfalle)    0,4 Gew.-%

Nach gaschromatographischer Analyse hat das Destillat folgende Zusammensetzung:

```
Lösungsmittel                                     1,5  Gew.-%

nicht identifizierte Komponenten                  5,9  Gew.-%

Safrol                                            0,3  Gew.-%

4-(3,4-Methylendioxyphenyl)-butanal              53,3  Gew.-%

2-Methyl-3-(4,5-Methylendioxy-
phenyl)-propanal                                 35,3  Gew.-%

2-(3,4-Methylendioxyphenyl)-butanal               2,4  Gew.-%

Nachlauf                                          1,3  Gew.-%
```

Bezogen auf reines Safrol beträgt die Ausbeute an Formylprodukten des Sassafrasöls 78,7 % der Theorie. Nach dem erfindungsgemäßen Verfahren hergestellte Hydroformylierungsprodukte von Sassafrasöl enthalten

0,1 bis 1       Gew.-% Safrol
0,1 bis 2,5      Gew.-% 2-(3,4-Methylendioxy-phenyl)-butanal
30 bis 40       Gew.-% 2-Methyl-3-(3,4-methylendioxyphenyl)-propanal
50 bis 60       Gew.-% 4-(3,4-Methylendioxyphenyl)-butanal
sowie bis 10     Gew.-% andere Hydroformylierungsprodukte von Sassafrasöl und gewünschtenfalls organische Lösungsmittel.

**Patentansprüche**

1. Verfahren zur Herstellung eines Riechstoffs, dadurch gekennzeichnet, daß man Sassafrasöl bei Drücken von 5 bis 50 MPa und Temperaturen von 70 bis 170°C in Gegenwart von Triphenylphosphan als Ligand enthaltenden Rhodiumcarbonyl-Komplexverbindungen zusammen mit überschüssigem Triphenylphosphan als Katalysator mit einem Gemisch aus Kohlenmonoxid und Wasserstoff umsetzt und das Reaktionsprodukt von Lösungsmittel und Katalysator abtrennt.

2. Verfahren nach Anspruch 1, dadurch gekennzeichnet, daß die Umsetzung bei 20 bis 30 MPa und 120 bis 140°C erfolgt.

3. Verfahren nach Anspruch 1 oder 2, dadurch gekennzeichnet, daß als Rhodiumcarbonyl-Komplexverbindung $HRhCO[(C_6H_5)_3P]_3$ eingesetzt wird.

4. Verfahren nach einem oder mehreren der Ansprüche 1 bis 3, dadurch gekennzeichnet, daß je g-At Rhodium 10 bis 150 Mol Triphenylphosphan vorliegen.

5. Verfahren nach einem oder mehreren der Ansprüche 1 bis 4, dadurch gekennzeichnet, daß Kohlenmonoxid und Wasserstoff im Volumverhältnis 1 : 4 bis 4 : 1, vorzugsweise 1 : 1, eingesetzt werden.

6. Hydroformylierungsprodukte von Sassafrasöl enthaltend

   | 0,1 bis 1 | Gew.-% Safrol |
   | 0,1 bis 2,5 | Gew.-% 2-(3,4-Methylendioxy-phenyl)-butanal |
   | 30 bis 40 | Gew.-% 2-Methyl-3-(3,4-methylendioxyphenyl)-propanal |
   | 50 bis 60 | Gew.-% 4-(3,4-Methylendioxyphenyl)-butanal |
   | sowie bis 10 | Gew.-% andere Hydroformylierungsprodukte von Sassafrasöl und gewünschtenfalls organische Lösungsmittel. |

7. Verwendung von Hydroformylierungsprodukten des Sassafrasöls als Riechstoff.

**Claims**

1. A process for the preparation of a fragrance, which comprises reacting sassafras oil under pressures from 5 to 50 MPa and at temperatures of 70 to 170°C in the presence of rhodium carbonyl complex compounds containing triphenylphosphine as ligand, together with excess triphenylphosphine as catalyst with a mixture of carbon monoxide and hydrogen, and removing solvent and catalyst from the reaction product.

2. The process as claimed in claim 1, wherein the reaction is carried out under 20 to 30 MPa and at 120 to 140°C.

3. The process as claimed in claim 1 or 2, wherein $HRhCO[(C_6H_5)_3P]_3$ is employed as rhodium carbonyl complex compound.

4. The process as claimed in one or more of claims 1 to 3, wherein 10 to 150 mol of triphenylphosphine are present per g at of rhodium.

5. The process as claimed in one or more of claims 1 to 4, wherein carbon monoxide and hydrogen are employed in the ratio 1 : 4 to 4 : 1, preferably 1 : 1, by volume.

6. Products of the hydroformylation of sassafras oil, containing

   | 0.1 to 1% | by weight of safrole |
   | 0.1 to 2.5% | by weight of 2-(3,4-methylenedioxyphenyl)butanal |
   | 30 to 40% | by weight of 2-methyl-3-(3,4-methylenedioxyphenyl)propanal |
   | 50 to 60% | by weight of 4-(3,4-methylenedioxyphenyl)butanal |
   | and up to 10% | by weight of other products of the hydroformylation of sassafras oil and, if required, organic solvents. |

7. The use of products of the hydroformylation of sassafras oil as fragrance.

**Revendications**

1. Procédé de préparation d'une matière odorante, caractérisé en ce que l'on fait réagir l'essence de sassafras à des pressions de 5 à 50 MPa et des températures de 70 à 170°C avec un mélange d'oxyde de carbone et d'hydrogène en présence d'un complexe carbonylé du rhodium contenant du triphénylphosphane en tant que ligand et d'un excès de triphénylphosphane, servant de catalyseur, et on débarrasse le produit de réaction du solvant et du catalyseur.

2. Procédé selon la revendication 1, caractérisé en ce que la réaction est effectuée à une pression de 20 à 30 MPa et une température de 120 à 140°C.

3. Procédé selon la revendication 1 ou 2, caractérisé en ce que le complexe carbonylé du rhodium est $HRhCO[(C_6H_5)_3P]_3$.

4. Procédé selon une ou plusieurs des revendications 1 à 3, caractérisé en ce qu'il y a de 10 à 150 mol de triphénylphosphane par atome-gramme de rhodium.

5. Procédé selon une ou plusieurs des revendications 1 à 4, caractérisé en ce que l'oxyde de carbone et l'hydrogène sont mis en oeuvre dans des proportions relatives en volume de 1 : 4 à 4: 1, de préférence de 1 : 1.

6. Produits d'hydroformylation de l'essence de sassafras, contenant:
   0,1 à 1 % en poids de safrole,
   0,1 à 2,5 % en poids de 2-(3,4-méthylènedioxyphényl)butanal,
   30 à 40 % en poids de 2-méthyl-3-(3,4-méthylènedioxyphényl)propanal,
   50 à 60 % en poids de 4-(3,4-méthylènedioxyphényl)butanal,
   et jusqu'à 10 % en poids d'autres produits d'hydroformylation de l'essence de sassafras et, si on le désire, de solvants organiques.

7. Utilisation des produits d'hydroformylation de l'essence de sassafras en tant que matières odorantes.